# EUROPEAN PATENT SPECIFICATION

(11) **EP 3 273 800 B1**
(45) Date of publication and mention of the grant of the patent: **18.03.2020**
(21) Application number: 16722934.3
(22) Date of filing: 18.03.2016
(51) Int. Cl.: A23L 3/28, A23B 7/015, A23B 9/06, A61L 2/10, B01J 19/12

(54) **AN APPARATUS AND A METHOD FOR TREATING PLANT PRODUCTS**
VORRICHTUNG UND VERFAHREN ZUR BEHANDLUNG VON PFLANZENPRODUKTEN
APPAREIL ET PROCÉDÉ DE TRAITEMENT DE PRODUITS VÉGÉTAUX

(30) Priority: 25.03.2015 IT MO20150068
(43) Date of publication of application: 31.01.2018
(73) Proprietor: Zavatti, Eleonora, 40053 Valsamoggia (Bologna) (IT)
(72) Inventor: ZAVATTI, Eleonora, 40053 Valsamoggia (Bologna) (IT); ZAVATTI, Federico, 40053 Valsamoggia (Bologna) (IT)
(74) Representative: Colò, Chiara
(86) International application number: PCT/IB2016/051527
(87) International publication number: WO 2016/151446

(56) References cited:
- WO-A1-03/077958
- DE-A1-102009 055 731
- GB-A- 2 388 764
- US-A1- 2009 098 259
- US-A1- 2009 311 392

## Description

The invention relates to an apparatus and a method for treating plant products, particularly ready-to-eat fruits and vegetables, also known to the experts from the sector as "fourth range products". More in detail, the apparatus and method according to the invention enables drying and decontamination of ready-for-use vegetables or fruit. Ready-to-eat plant products such as fruits and vegetables packed in special packets or in sealed containers already cleaned and washed, are having considerable success in the fruit and vegetables market. These products are appreciated due to the fact that they may be promptly consumed without the end user having to clean or wash them which would result in time wasting.

The process for the preparation of ready-to-eat fruit or vegetables provides a selection step and a sorting step, followed by a step of cutting the products into ready-to-eat suitably sized pieces. Fruit or vegetables are then washed, dried off and finally packed in sealed packets or containers. The ready-to-eat plant products shall be kept at a temperature below 8°C from the moment they leave the manufacturing plant, until the moment in which they are exposed in refrigerated displays of supermarkets or generally speaking in stores. When washing fruits or vegetables intended to be sold as ready-to eat-products, use is made of chemically treated washing water to which detergent-sanitizers additives were added for the purposes of testing the microbial load of fruits or vegetables. Special regulations are of course foreseen which set forth use of the previously mentioned additives, in order that any side effects arising therefrom are minimized. In Italy chlorinated solutions containing 80-100 ppm chlorine are generally used as washing water.

A drawback of the known processes for the preparation of ready-to-eat plant products, is that the chemically treated washing water leaves residues thereon.

It was proved that the most used sanitizing agents, namely chlorine or related compounds, may result in the formation of hazardous residues on foods in general. Some researches, including a Japanese research from the University Shizuoka, even hypothesize that these residues might give rise to carcinogenic effects. Indeed, when exposed to chlorinated products formed in drinking water, natural organic substances of foods react, thus forming carcinogenic compounds termed by Japanese researchers "MX", i.e. unknown mutagens.

This effect is particularly marked in the case of ready-to-eat plant products which are generally not subject to household washings with water having a relatively low content in chlorine, which would promote dilution of the hazardous compounds originated from industrial washings with chemically treated water with consequent reduction of risks to health.

A further drawback which may occur with ready-to-eat plant products is that, after having being packaged, they tend to deteriorate rapidly, thus acquiring a non-inviting appearance. These products are therefore discarded by consumers, who prefer to purchase fruits or vegetables packages with a fresher appearance. This results in considerable wastage of fruits and vegetables.

Drying machines are known which are aimed at drying plant products intended to be sold as ready-to-eat products, so as to remove the washing water with which such products were washed. Drying machines of the known type are substantially of the centrifugal type, or provided with drainage bands, or still of the tunnel type.

Although the drying machines of the known type are able to dry off plant products they are interacting with, their effectiveness can be definitively improved.

*Known related apparatuses and applications are described in patents* WO03077958*,* DE 102009055731*,* US2009311392*,* GB2388764 *and* US2009098259*.*

It is an object of the invention to improve the processing of the so-called fourth range plant products, particularly fruits and vegetables, i.e. the ready-to-eat sold products, wherein the following aims are attained:
- reducing the health risks taken by consumers when purchasing and consuming ready-to-eat fruits and vegetables, thereby reducing the amount of chemical residues left on ready-to-eat plant products after washing;
- increasing freshness of ready-for-use plant products, thereby extending their life once exposed on the refrigerated counters of supermarkets;
- improving effectiveness of the drying machines suitable for drying off plant products intended to be sold as ready-to-eat products after that the same were washed with washing water.

In a first aspect of the invention, an apparatus is provided for treating ready-for-use plant products, which apparatus comprises a treatment chamber for receiving the plant products, sanitizing means for superficially decontaminating the plant products, characterized in that the sanitizing means comprises at least one UV-C radiation source for emitting UV-C rays directly onto the plant products present inside the treatment chamber.

The UV-C rays emitted directly onto the plant products inside the treatment chamber, exert an effective germicidal action on such products, thereby making it possible to eliminate - or at worst to minimize - use of chemical sanitizers additives such as chlorine and compounds thereof. Hence, it is drastically reduced or even eliminated the amount of potentially hazardous compounds to the health which are formed on ready-for-use plant products and generated by the residues remaining on such products after that the same were washed with chemically treated water.

The removal (or reduction in concentration) of chlorine in the washing water, further ensures compliance with the statutory limit on the concentration of chlorine in the wastewater which is to be discharged into the drains.

In some Countries this limit is very restrictive. To cite an example, in Italy it is not more than 3 mg/liter.

Furthermore, the UV-C radiation on plant products gives rise to a phenomenon known as "hormesis", which allows to stimulate beneficial reactions on the biological structures of plant products. It is particularly stimulated the production of certain enzymes, such as flavonoids, phytoalexins and the like, which are microorganisms inhibitors and protective for the plant. This allows the plant to activate its natural defenses even after that the products thereof were collected.

This makes plant products more resistant, thereby extending the life thereof when exposed on refrigerated counters of supermarkets.

In one embodiment, the apparatus comprises a drying system associated with the treatment chamber for drying the plant products while the plant products are being treated by said at least one UV-C radiation source.

Owing to the drying system, the particles of washing water can be dried off, which particles remained on the plant products previously washed. Since the drying system is provided in the treatment chamber, the plant products can be dried off while being sanitized via UV-C rays, which allows to optimize the production times. The drying system comprises at least one ventilation device intended to send an air flow onto the plant products present inside the treatment chamber.

In particular, the ventilation device has an outlet mouth flowing into a lower region of the treatment chamber.

The air flow coming out from the ventilation device causes the plant products inside the treatment chamber to move with a turbulent motion, which allows the washing water to evaporate in an effective manner allowing at same time the UV-C rays to radiate almost the entire surface of the plant products in a uniform manner.

In a second aspect of the invention, a method is provided for obtaining ready-for-use plant products comprising the steps of washing the plant products and decontaminating superficially the plant products, characterized in that, during the step of decontaminating, the plant products are irradiated with UV-C rays. By the term "decontaminating step" it is meant a microbiological decontamination mainly aimed at attacking fungi, bacteria, yeasts and molds. The plant products are dried off and irradiated with UV-C rays.

In particular, the plant products are dried off with an air flow being directed from the bottom into a treatment chamber, wherein said plant products are irradiated with UV-C rays.

Owing to the method provided by the second aspect of the invention, it is possible to prevent the surface of plant products from being subject to the formation of harmful compounds deriving from substances present in the washing water, or at least to limit the quantity thereof. Owing to the hormesis process and to the germicidal action induced by the UV-C rays, it is further possible to render plant products more resistant. This allows to extend the freshness time of plant products.

The invention will be better understood and implemented with reference to the appended figures showing a non-limiting example thereof, in which:

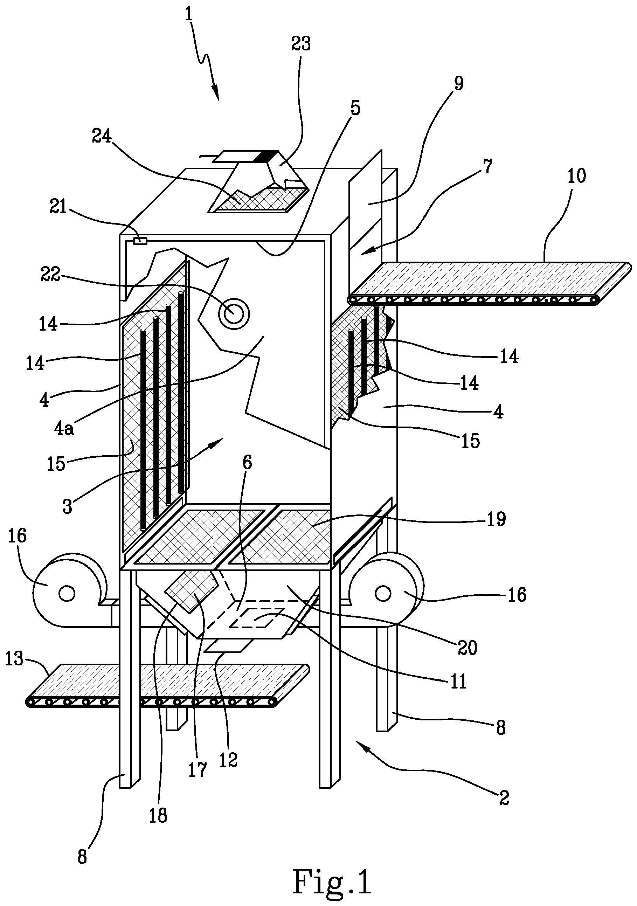

Figure 1 is a schematic perspective view of an apparatus for treating plant products.

Figure 1 shows an apparatus 1 for treating plant products, particularly vegetable or fruit, usable during a process aimed at obtaining the so-called "fourth range" plant products i.e. ready-to-eat products.

As will be better described hereinafter, the apparatus 1 allows to sanitize and dry off plant products that have been previously washed with washing water. In order to obtain ready-to-use plant products, the apparatus 1 may therefore be positioned in a production line downstream of a washing machine, wherein the plant products are subjected to a washing process aimed at eliminating the coarsest decontaminating substances, such as potting soil, from their surface. Located downstream of the apparatus 1, a packaging machine may be provided for packaging the plant products treated by the apparatus 1 in plastic wrappings or other sealed packages.

Alternatively the apparatus may be suitably sized and designed for domestic use.

The apparatus 1 comprises a support frame 2 which supports a treatment chamber 3 suitable to receive the plant products. The support frame 2 may comprise a plurality of support feet 8, for example four support feet 8, on which the treatment chamber 3 is mounted. As it will be better described in a later section, the plant products in the treatment chamber 3 are subjected to a drying step and simultaneously to a disinfecting or sanitizing step, aimed at breaking down the microbial load present on the surface of the plant products.

The treatment chamber 3 may be substantially parallelepiped-shaped. In the example shown, the treatment chamber 3 is delimited by a plurality of side walls 4, for example arranged vertically, and by an upper wall 5, which can be substantially horizontal. The treatment chamber 3 further has a base 6 which is disposed opposite to the upper wall 5.

The treatment chamber 3 is provided with an inlet opening 7 through which the plant products to be treated can enter the treatment chamber 3. The inlet opening 7 may be obtained on a side wall 4, in particular in an upper region of the treatment chamber 3. The apparatus 1 comprises an inlet door 9 associated with the inlet opening 7, which inlet door 9 may be opened or closed in a programmable manner, in order to allow the plant products to enter into the treatment chamber 3 or, respectively, to isolate the treatment chamber 3 from the outside while the plant products are being dried off and sanitized.

An inlet conveyor device 10, for example of the belt type, may be provided for conveying the plant products to be treated towards the inlet opening 7.

The treatment chamber 3 is provided with an outlet opening 11 through which the plant products may leave the treatment chamber 3 after having being sanitized and dried off. The outlet opening 11 may be formed on the base 6 of the treatment chamber 3. To the outlet opening 11 an outlet door 12 is associated which may be opened or closed in a programmable manner.

An outlet conveyor device 13, for example of the belt type, may be arranged in proximity of the outlet opening 11 in order to remove the already treated plant products from the apparatus 1.

The apparatus 1 comprises sanitizing means to decontaminate superficially the plant products which are present inside the treatment chamber 3. The sanitizing means comprises at least one UV-C radiation source for emitting UV-C rays directly onto the plant products present inside the treatment chamber. In the example illustrated, the sanitizing means comprises a plurality of UV-C radiation sources 14, each of which is shaped as a UV-C lamp, the UV-C radiation sources 14 being associated to the side walls 4 of the treatment chamber 3.

The UV-C rays consist in an electromagnetic radiation having a wavelength which falls within a particular range of wavelengths characterizing ultraviolet rays. This range is roughly between 100 and 280 nm. The UV-C radiation are further known as germicidal ultraviolet radiation, as they are able to modify the DNA or the RNA of the microorganisms, thereby preventing them from reproducing or being harmful. When the UV-C rays come in contact with the plant products, the UV-C rays exert a disinfection action, thus reducing the microbial load on plant products after the latter have been washed.

In the example herein described, each UV-C radiation source 14 is of the diffuse type, i.e. of the non-consistent type, and emits rays within the entire UV-C spectrum with a prevailing wavelength around 254 nm.

Each UV-C radiation source may be shaped as an elongated body extending predominantly along a direction of extension. In the example shown, the UV-C radiation sources 14 are positioned in such a manner that each UV-C radiation source 14 extends predominantly along a vertical direction.

In the embodiment shown in Figure 1, the UV-C radiation sources 14 are associated with two side walls 4 opposite to one another. This configuration allows to notably reduce failure to irradiation due to shading phenomena between the plants.

It is further possible to provide UV-C radiation sources 14 on a walls number of the treatment chamber 3 other than two, for example on more than two side walls 4 of the treatment chamber 3.

Theoretically the UV-C radiation source 14 may be further associated to those walls of the treatment chamber 3 which are different from the side walls 4, for example to the upper wall 5.

The UV-C radiation sources 14 are anchored to the side walls 4 of the treatment chamber 3 by means of anchoring elements of the known type.

Protection grids 15 may be further provided, which are interposed between the UV-C radiation sources 14 and the inside of the treatment chamber 3, and which exert a protective function for UV-C radiation sources 14.

The apparatus 1 further comprises a drying system for drying off the plant products present inside the treatment chamber 3 while the plant products are being irradiated with UV-C rays emitted by the sources 14.

The drying device comprises at least a ventilation device 16 which emits an air flow inside the treatment chamber 3. In the example illustrated, there are provided two ventilation devices 16.

The ventilation devices 16 are associated with the base 6 of the treatment chamber 3 in such a manner as to emit respective air flows which are direct from the bottom upwards. To this end, the ventilation devices 16 have respective outlet mouths 17 from each of which an air flow is coming out, which is flowing onto the basis 6 of the treatment chamber 3. In Figure 1 a single outlet mouth 17 is visible.

The outlet mouths 17 may be protected by respective grids 18. The air flow emitted from the ventilation devices 16 cause the washing water still present on plant products to evaporate. Furthermore, the air flows emitted by the ventilation devices 16 give rise to a turbulent motion of the plant products present inside the treatment chamber, which promotes evaporation of the washing water besides improving the exposure of plant products to UV-C rays emitted by the sources 14. In this way, the plant products may be sanitized with great effectiveness in that the whole surface of the plant products may be substantially irradiated by the UV-C radiation sources 14.

An auxiliary wall 19 may be arranged in the treatment chamber 3 above the base 6, in order that the plant products are confined within a space located close to the UV-C radiation sources 14.

The auxiliary wall 19 may be provided with a grid-shaped structure. The auxiliary wall 19 is movable in a programmed manner between a closed position shown in Figure 1, and an open position not shown.

In the closed position, the auxiliary wall 19 prevents the plant products contained inside the treatment chamber 3 from falling, up to reaching the base 6. In the closed position, between the auxiliary wall 19 and the base 6, an empty volume 20 is defined, wherein the outlet mouths 17 of the ventilation devices 16 are opening. The plant products being absent from the empty volume 20. The air emitted by the ventilation devices 16 into the empty volume 20 passes through the auxiliary wall 19, which auxiliary wall 19, as already mentioned, may be provided with a grid-shaped structure or otherwise with openings and reaches the plant products contained in the treatment chamber 3.

In the open position of the auxiliary wall 19, the plant products are falling onto the base 6 so as to exit from the apparatus 1.

One of the side walls 4 which delimit the treatment chamber 3 may be conformed as an openable door 4a through which an operator may enter the treatment chamber 3, for example for cleaning, maintenance or inspection purposes.

To the openable door 4a, a safety element 21 is associated, for example a micro-switch which switches off the UV-C radiation sources 14 and disables operation of the apparatus 1 in the event that the openable door 4a is accidentally opened while the plant products are being treated inside the treatment chamber 3. This prevents the UV-C rays from causing damages to operators in case the openable door 4a is accidentally opened.

The openable door 4a may be provided with a control window 22 of transparent material and yet shielding against UV-C rays, which control window 22 is particularly shaped as a porthole, through which an operator can safely control operation of the apparatus 1 while the plant products are being treated.

A hood 23 is formed on the upper wall 5 to allow the water vapor which develops inside the treatment chamber 3 to exit from the apparatus 1. The hood 23 may be opened or closed in a programmable manner. On the upper wall 5, in proximity of the hood 23, a grid element 24 is disposed in order to prevent the plant products present inside the treatment chamber 3 from exiting from the apparatus 1.

The structure and mechanical components of the apparatus 1 are made of stainless steel for direct food contact.

The apparatus 1, and in particular the parts thereof facing the treatment chamber 3, are arranged within the latter and washable with running water. To this end, the electrical components of the apparatus 1 are designed with a proper degree of protection.

During operation, the plant products already washed are conveyed towards the apparatus 1 by the inlet conveyor device 10. The inlet door 9 is open and the plant products conveyed by the inlet conveyor device 10, are falling from the top downwards due to gravity, into the treatment chamber 3 by passing through the inlet opening 7.

The auxiliary wall 19 is in its closed position, so that the plant products cannot fall onto the base 6, but remain in the region of the treatment chamber 3 wherein the UV-C radiation sources 14 are present.

Once a desired amount of plant products has entered inside the treatment chamber 3, the inlet door 9 is closed and the inlet conveyor device 10 is stopped.

The UV-C radiation sources 14 begin emitting UV-C rays, which radiate directly the plant products present inside the treatment chamber 3.

At the same time, the ventilation devices 16 emit respective air flows in order to dry off the plant products, thereby removing the residues of washing water still present on their surface. The air flows emitted by the ventilation devices are directed from the bottom upwards, which gives rise to high turbulence inside the treatment chamber 3, so that evaporation of washing water is promoted and disinfection of plant products via UV-C rays is made highly effective. In fact, by moving the plant products inside the treatment chamber 3 in a turbulent manner, the entire surface of such plant products comes to be substantially exposed to UV-C rays, which results in a particularly effective germicidal action.

Besides exerting a germicidal action on the plant products present inside the treatment chamber 3, the UV-C rays gives rise to an hormesis process on plant products, owing to which - as previously mentioned - the life of plant products is extended.

Inside the treatment chamber 3, the plant products are irradiated with UV-C rays according to a treatment time and a treatment intensity, which can be set by the operator on the basis of the type of plant products which are being treated, i.e. according to the fruit or vegetable varieties that the apparatus 1 is processing.

An average treatment time may be in the order of a few minutes. UV-C rays are delivered at low doses, particularly at doses of 0.25-8.0 kJ/m².

This dosage is particularly effective for simultaneously ensuring the germicidal action and the hormesis process without the plant products being damaged.

Once the processing of plant products inside the treatment chamber comes to an end, the auxiliary wall 19 moves into the open position, so that the plant products are made to fall onto the base 6.

It is then opened the outlet door 12, so that the already treated plant products may leave the apparatus 1 by coming out from the outlet opening 11 due to gravity. The plant products fall onto the outlet conveyor device 13 through the outlet opening 11, which conveyor device 13 transports the plant products towards subsequent working stations, such as for example packaging machines destined to pack them inside suitable packages that consumers may purchase when they wish ready-to-eat plant products.

## Claims

1. A method for obtaining ready-for-use plant products, comprising the steps of washing and superficially decontaminating the plant products, wherein, during the decontaminating step, the plant products are irradiated with UV-C rays in a treatment chamber (3), **characterized in that** the plant products are dried by an air flow coming out of a ventilation device while they are being irradiated with UV-C rays, the air flow being sent in the treatment chamber (3) from the bottom upwards thereby causing the plant products to move with a turbulent motion in the treatment chamber (3).

2. A method according to claim 1, wherein, during the decontaminating step, the plant products are irradiated with UV-C radiation in doses of 0.25-8.0 kJ/m², preferably for a treatment time of a few minutes.

3. A method according to claim 1 or 2, wherein said decontaminating step is of a microbiological nature and mainly addressed to fungi, bacteria, yeasts and molds.

4. An apparatus for treating ready-for-use plant products, comprising a treatment chamber (3) for receiving the plant products, sanitizing means for superficially decontaminating the plant products, the sanitizing means comprising at least one UV-C radiation source (14) for emitting UV-C rays directly onto the plant products inside the treatment chamber (3), said apparatus further comprising a drying system associated with the treatment chamber (3) for drying the plant products while the plant products are being sanitized by said at least one UV-C radiation source (14), **characterized in that** the drying system comprises at least one ventilation device intended to send an air flow onto the plant products present inside the treatment chamber at the same time as said at least one UV-C radiation source (14) emits UV-C rays, said at least one ventilation device (16) being configured to emit an air flow directed from the bottom upwards into the treatment chamber (3) so that the air flow coming out from the ventilation device causes the plant products inside the treatment chamber to move with a turbulent motion.

5. An apparatus according to claim 4, wherein said at least one ventilation device (16) has an outlet mouth (17) for emitting said air flow, the outlet mouth (17) being provided on a base (6) of the treatment chamber (3).

6. An apparatus according to claim 5, and further comprising an auxiliary wall (19) arranged in the treatment chamber (3) for holding the plant products close to said at least one UV-C radiation source (14), the auxiliary wall (19) being movable between a closed position and an open position, and wherein, in the closed position, an empty volume (20) is defined between the base (6) and the auxiliary wall (19), the plant products being absent from the empty volume (20), whereas in the open position the auxiliary wall (19) allows the plant products to fall onto the base (6).

7. An apparatus according to any one of claims 4 to 6, wherein said at least one UV-C radiation source (14) is provided on a side wall (4) of the treatment chamber (3).

8. An apparatus according to any one of claims 4 to 7, wherein a plurality of UV-C radiation sources (14) is provided, said UV-C radiation sources (14) being positioned on at least two opposite side walls (4) of the treatment chamber (3), said UV-C radiation sources (14) being preferably shaped as elongated bodies extending along a substantially vertical direction.

9. An apparatus according to any one of claims 4 to 8, wherein said at least one UV-C radiation source (14) is a diffuse type source, capable of emitting radiation within the full UV-C spectrum, with a prevailing wavelength of about 254 nm.

10. An apparatus according to any one of claims 4 to 9, wherein the treatment chamber (3) is provided with an inlet opening (7) which can be selectively opened and closed for enabling the plant products that are to be treated to enter the treatment chamber (3), the treatment chamber (3) being further provided with an outlet opening (11) which can be selectively opened and closed for allowing the treated plant products to leave the apparatus (1), the inlet opening (7) being obtained in an upper portion of the treatment chamber (3), the outlet opening (11) being obtained in a lower portion of the treatment chamber (3).

11. An apparatus according to any one of claims 4 to 10, wherein the treatment chamber (3) is provided with an inlet opening (7) through which the plant products to be treated can enter the treatment chamber (3), the apparatus (1) comprising an inlet door (9) associated with the inlet opening (7), said inlet door (9) may be opened or closed in a programmable manner, in order to allow the plant products to enter into the treatment chamber (3) or, respectively, to isolate the treatment chamber (3) from the outside while the plant products are being dried off and sanitized, an inlet conveyor device (10) being provided for conveying the plant products to be treated towards the inlet opening (7).

## Patentansprüche

1. Verfahren zum Erhalten gebrauchsfertiger Pflanzenprodukte, umfassend die Schritte des Waschens und des oberflächlichen Dekontaminierens der Pflanzenprodukte, wobei die Pflanzenprodukte, während des Schrittes des Dekontaminierens, in einer Behandlungskammer (3) mit UV-C-Strahlen bestrahlt werden, **dadurch gekennzeichnet, dass** die Pflanzenprodukte durch einen aus einer Belüftungsvorrichtung austretenden Luftstrom getrocknet werden, während sie mit UV-C-Strahlen bestrahlt werden, wobei der Luftstrom von unten nach oben in die Behandlungskammer (3) eingeleitet wird, wodurch bewirkt wird, dass sich die Pflanzenprodukte mit einer turbulenten Bewegung in der Behandlungskammer (3) bewegen.

2. Verfahren nach Anspruch 1, wobei, während des Schrittes des Dekontaminierens, die Pflanzenprodukte mit UV-C-Strahlung in Dosierungen von 0,25-8,0 kJ/m² bestrahlt werden, vorzugsweise für eine Behandlungsdauer von einigen Minuten.

3. Verfahren nach Anspruch 1 oder 2, wobei der Schritt des Dekontaminierens mikrobiologischer Natur ist und hauptsächlich auf Pilze, Bakterien, Hefen und Schimmelpilze abzielt.

4. Vorrichtung zur Behandlung gebrauchsfertiger Pflanzenprodukte, umfassend eine Behandlungskammer (3) zur Aufnahme der Pflanzenprodukte, Hygieneeinrichtungen zum oberflächlichen Dekontaminieren der Pflanzenprodukte, wobei die Hygieneeinrichtungen zumindest eine UV-C-Strahlungsquelle (14) zum Aussenden von UV-C-Strahlen direkt auf die Pflanzenprodukte in der Behandlungskammer (3) umfassen, wobei die Vorrichtung ferner ein Trocknungssystem umfasst, das mit der Behandlungskammer (3) verbunden ist, um die Pflanzenprodukte zu trocknen, während die Pflanzenprodukte durch die zumindest eine UV-C-Strahlungsquelle (14) hygienisch gereinigt werden, **dadurch gekennzeichnet, dass** das Trocknungssystem zumindest eine Belüftungsvorrichtung umfasst, die dafür bestimmt ist, einen Luftstrom auf die in der Behandlungskammer vorhandenen Pflanzenprodukte einzuleiten, während gleichzeitig die zumindest eine UV-C-Strahlungsquelle (14) UV-C-Strahlen aussendet, wobei die zumindest eine Belüftungsvorrichtung (16) dafür ausgelegt ist, einen von unten nach oben gerichteten Luftstrom in die Behandlungskammer (3) einzuleiten, so dass der aus der Belüftungsvorrichtung austretende Luftstrom bewirkt, dass sich die Pflanzenprodukte in der Behandlungskammer mit einer turbulenten Bewegung bewegen.

5. Vorrichtung nach Anspruch 4, wobei die zumindest eine Belüftungsvorrichtung (16) eine Auslassmündung (17) zur Ausgabe des Luftstroms aufweist, wobei die Auslassmündung (17) auf einer Basis (6) der Behandlungskammer (3) vorgesehen ist.

6. Vorrichtung nach Anspruch 5, und ferner umfassend eine Hilfswand (19) die in der Behandlungskammer (3) angeordnet ist, um die Pflanzenprodukte nahe der zumindest einen UV-C-Strahlungsquelle (14) zu halten, wobei die Hilfswand (19) beweglich ist zwischen einer geschlossenen Stellung und einer offenen Stellung, und wobei, in der geschlossenen Stellung, ein Leervolumen (20) zwischen der Basis (6) und der Hilfswand (19) definiert wird, wobei die Pflanzenprodukte sich nicht im Leervolumen (20) befinden, während in der offenen Stellung die Hilfswand (19) das Herabfallen der Pflanzenprodukte auf die Basis (6) ermöglicht.

7. Vorrichtung nach einem der Ansprüche von 4 bis 6, wobei die zumindest eine UV-C-Strahlungsquelle (14) auf einer Seitenwand (4) der Behandlungskammer (3) vorgesehen ist.

8. Vorrichtung nach einem der Ansprüche von 4 bis 7, wobei eine Vielzahl von UV-C-Strahlungsquellen (14) vorgesehen ist, wobei die UV-C-Strahlungsquellen (14) auf zumindest zwei einander gegenüberliegenden Seitenwänden (4) der Behandlungskammer (3) angeordnet sind, und die UV-C-Strahlungsquellen (14) vorzugsweise in Gestalt von länglichen Körpern geformt sind, die sich entlang einer im Wesentlichen vertikalen Richtung erstrecken.

9. Vorrichtung nach einem der Ansprüche von 4 bis 8, wobei die zumindest eine UV-C-Strahlungsquelle (14) eine diffuse Strahlungsquelle ist, die in der Lage ist, Strahlung innerhalb des gesamten UV-C-Spektrums mit einer vorherrschenden Wellenlänge von ungefähr 254 nm auszusenden.

10. Vorrichtung nach einem der Ansprüche von 4 bis 9, wobei die Behandlungskammer (3) eine Einlassöffnung (7) aufweist, die selektiv geöffnet und geschlossen werden kann, um das Eintreten der zu behandelnden Pflanzenprodukte in die Behandlungskammer (3) zu ermöglichen, wobei die Behandlungskammer (3) ferner eine Auslassöffnung (11) aufweist, die selektiv geöffnet und geschlossen werden kann, um zu ermöglichen, dass die behandelten Pflanzenprodukten die Vorrichtung (1) verlassen können, wobei die Einlassöffnung (7) in einem oberen Abschnitt der Behandlungskammer (3) und die Auslassöffnung (11) in einem unteren Abschnitt der Behandlungskammer (3) verwirklicht ist.

11. Vorrichtung nach einem der Ansprüche von 4 bis 10, wobei die Behandlungskammer (3) eine Einlassöffnung (7) aufweist, durch welche die zu behandelnden Pflanzenprodukte in die Behandlungskammer (3) eintreten können, wobei die Vorrichtung (1) eine Einlasstür (9) umfasst, die mit der Einlassöffnung (7) verbunden ist, wobei die Einlasstür (9) in programmierbarer Weise geöffnet und geschlossen werden kann, um das Eintreten der Pflanzenprodukte in die Behandlungskammer (3) zu ermöglichen, beziehungsweise um die Behandlungskammer (3) von der Außenumgebung zu isolieren, während die Pflanzenprodukte getrocknet und hygienisch gereinigt werden, wobei eine Einlassfördervorrichtung (10) dafür vorgesehen ist, die zu behandelnden Pflanzenprodukte zu der Einlassöffnung (7) zu befördern.

## Revendications

1. Un procédé pour obtenir des produits végétaux prêts à l'emploi, comprenant les phases consistant à laver et à décontaminer superficiellement les produits végétaux, où, pendant la phase de décontamination, les produits végétaux sont irradiés avec des rayons UV-C dans une chambre de traitement (3), **caractérisé en ce que** les produits végétaux sont séchés par un flux d'air provenant d'un dispositif de ventilation alors qu'ils sont irradiés avec les rayons UV-C, le flux d'air étant envoyé dans la chambre de traitement (3) du bas vers le haut entraînant ainsi les produits végétaux à se déplacer avec un mouvement turbulent dans la chambre de traitement (3).

2. Le procédé selon la revendication 1, dans lequel, pendant la phase de décontamination, les produits végétaux sont irradiés avec un rayonnement UV-C dans des doses de 0,25-8,0 kJ/m², de préférence pendant un temps de traitement de quelques minutes.

3. Le procédé selon la revendication 1 ou 2, dans lequel ladite phase de décontamination est de nature microbiologique et concerne principalement les champignons, bactéries, levures et moisissures.

4. Un appareil de traitement de produits végétaux prêts à l'emploi, comprenant une chambre de traitement (3) pour recevoir les produits végétaux, des moyens de désinfection pour décontaminer superficiellement les produits végétaux, les moyens de désinfection comprenant au moins une source de rayonnement UV-C (14) pour émettre des rayons UV-C directement sur les produits végétaux à l'intérieur de la chambre de traitement (3), ledit appareil comprenant en outre un système de séchage associé à la chambre de traitement (3) pour sécher les produits végétaux alors que les produits végétaux sont désinfectés par ladite au moins une source de rayonnement UV-C (14), **caractérisé en ce que** le système de séchage comprend au moins un dispositif de ventilation destiné à envoyer un flux d'air sur les produits végétaux présents à l'intérieur de la chambre de traitement en même temps que ladite au moins une source de rayonnement UV-C (14) émet les rayons UV-C, ledit au moins un dispositif de ventilation (16) étant configuré pour émettre un flux d'air dirigé du bas vers le haut dans la chambre de traitement (3) de manière à ce que le flux d'air sortant du dispositif de ventilation entraîne les produits végétaux à l'intérieur de la chambre de traitement à se déplacer avec un mouvement turbulent.

5. L'appareil selon la revendication 4, dans lequel ledit au moins un dispositif de ventilation (16) a une bouche de sortie (17) pour émettre ledit flux d'air, la bouche de sortie (17) étant prévue sur une base (6) de la chambre de traitement (3).

6. L'appareil selon la revendication 5, et comprenant en outre une paroi auxiliaire (19) disposée dans la chambre de traitement (3) pour maintenir les produits végétaux près de ladite au moins une source de rayonnement UV-C (14), la paroi auxiliaire (19) étant mobile entre une position fermée et une position ouverte, et dans lequel, dans la position fermée, un volume vide (20) est défini entre la base (6) et la paroi auxiliaire (19), les produits végétaux étant absents du volume vide (20), tandis que dans la position ouverte la paroi auxiliaire (19) permet aux produits végétaux de tomber sur la base (6).

7. L'appareil selon l'une quelconque des revendications de 4 à 6, dans lequel ladite au moins une source de rayonnement UV-C (14) est prévue sur une paroi latérale (4) de la chambre de traitement (3) .

8. L'appareil selon l'une quelconque des revendications de 4 à 7, dans lequel une pluralité de sources de rayonnement UV-C (14) est prévue, lesdites sources de rayonnement UV-C (14) étant positionnées sur au moins deux parois latérales (4) opposées de la chambre de traitement (3), lesdites sources de rayonnement UV-C (14) étant de préférence conformées comme des corps allongés s'étendant le long d'une direction essentiellement verticale.

9. L'appareil selon l'une quelconque des revendications de 4 à 8, dans lequel ladite au moins une source de rayonnement UV-C (14) est une source de type diffus, capable d'émettre un rayonnement dans tout le spectre UV-C, avec une longueur d'onde dominante de 254 nm environ.

10. L'appareil selon l'une quelconque des revendications de 4 à 9, dans lequel la chambre de traitement (3) est dotée d'une ouverture d'entrée (7) qui peut être sélectivement ouverte et fermée pour permettre aux produits végétaux devant être traités d'entrer dans la chambre de traitement (3), la chambre de traitement (3) étant en outre dotée d'une ouverture de sortie (11) qui peut être sélectivement ouverte et fermée pour permettre aux produits végétaux traités de quitter l'appareil (1), l'ouverture d'entrée (7) étant obtenue dans une portion supérieure de la chambre de traitement (3), l'ouverture de sortie (11) étant obtenue dans une portion inférieure de la chambre de traitement (3).

11. L'appareil selon l'une quelconque des revendications de 4 à 10, dans lequel la chambre de traitement (3) est dotée d'une ouverture d'entrée (7) à travers laquelle les produits végétaux à traiter peuvent entrer dans la chambre de traitement (3), l'appareil (1) comprenant une porte d'entrée (9) associée à l'ouverture d'entrée (7), ladite porte d'entrée (9) peut être ouverte ou fermée de façon programmable, de manière à permettre aux produits végétaux d'entrer dans la chambre de traitement (3) ou, respectivement, à isoler la chambre de traitement (3) par rapport à l'extérieur alors que les produits végétaux sont séchés et désinfectés, un dispositif transporteur d'entrée (10) étant prévu pour transporter les produits végétaux à traiter vers l'ouverture d'entrée (7).
